# EUROPEAN PATENT APPLICATION

(11) **EP 4 005 624 A1**
(43) Date of publication of application: **01.06.2022**
(21) Application number: 21207720.0
(22) Date of filing: 11.11.2021
(51) Int. Cl.: A61M 27/00

(54) **APPARATUS FOR DRAINING LYMPH INTO VEINS**

(30) Priority: 12.11.2020 TW 109139556
(71) Applicant: Cheng, Christine Genlone, Taoyuan City 333 (TW)
(72) Inventor: CHENG, Ming Huei, Taipei City 114 (TW)
(74) Representative: Ferreccio, Rinaldo

(57) **Abstract**

An apparatus for draining lymph into vein includes a drainage device 2 which includes a chamber 21, an osmosis membrane 22, a first catheter 231, a second sensing element 25, and a pump 24. The chamber includes a chamber wall, wherein a chamber space is formed by the chamber wall, and a first opening is formed on the chamber wall. The osmosis membrane disposed on the first opening. One end of the catheter connects to the chamber space, and the other end of the catheter connects with a vein. The second sensing element receives a radio wave emitted by a first sensing element of a power supply device to send an electrical signal. The pump is disposed in the chamber and connecting to the second sensing element, wherein a negative pressure in the chamber space is generated by the pump to drain lymph into the vein via the first catheter.

## Description

### CROSS REFERENCE TO RELATED APPLICATION

This application claims the benefits of the Taiwan Patent Application Serial Number 109139556, filed on November 12, 2020, the subject matter of which is incorporated herein by reference.

### BACKGROUND OF THE INVENTION

### 1. Field of the Invention

The present invention relates to an apparatus for draining lymph into veins and, more particularly, to a novel apparatus for draining lymph suitable for effectively relieving lymphedema.

### 2. Description of Related Art

Lymphedema refers to swelling caused by the accumulation of excessive lymph in body tissues. Under normal circumstances, 10% of the arterial blood exuded from the capillaries contains protein-rich lymph that flows through the lymphatic vessels to the lymph nodes, and flows back to the veins in the end. However, under conditions that the proximal lymph nodes are surgically removed, or that the function of the remaining lymph nodes is destroyed due to radiotherapy, or that the lymphatic system is naturally lost, or that parasites destroy the lymphatic system, it is difficult to recycle the lymph leading to its accumulation in tissues resulting in lymphedema.

Generally, breast cancer patients are more prone to upper extremity lymphedema. According to statistics, about 6-30% of breast cancer patients will develop lymphedema, because breast cancer is usually treated with surgery or radiotherapy. The axillary lymphatic system is removed in the surgery treatment, and the radiotherapy makes remaining lymphatic tissues become fibrotic. Some of the breast cancer patients develop lymphedema within 3 years after receiving radiation or surgery. Because lymphedema is an accumulation of the excessive tissue fluid containing high osmotic pressure protein in the subcutaneous fat, it is easy to grow bacteria and show the swelling in appearance. In severe cases, it leads to skin lesions, fibrosis and cellulitis, thereby affecting daily life and appearance, and even threatening life.

Nowadays, the clinical treatment for lymphedema is mainly through extremity compression, massage, rehabilitation or wearing compression garments to reduce the edema level; and, cellulitis or lymphangitis is prevented and treated with medication. However, for patients with severe lymphedema, surgical intervention or implantation of lymphatic drainage apparatus in the subcutaneous tissue is required to treat lymphedema in a more aggressive manner. All the conventional lymph drainage devices have batteries placed inside the device to provide the power source for the lymph drainage device to operate. However, the built-in battery may not only run out of power, but also leak out its internal substances over time. Therefore, patients need to receive periodic surgeries to replace the built-in battery, which causes many inconveniences in patients' lives and affects their quality of life.

Therefore, in order to solve the aforementioned problems, it is urgent to develop an improved lymph drainage device to eliminate or alleviate the aforementioned problems.

### SUMMARY OF THE INVENTION

In view of this, the present invention provides an apparatus for draining lymph into veins to reduce the number of operations or solve problems such as the possible collapse of the built-in battery.

In order to achieve the aforementioned objective, an apparatus for draining lymph into veins, comprising a drainage device is provided. The drainage device comprises a chamber, an osmosis membrane, a first catheter, a second sensing element, and a pump. The chamber comprises a chamber wall forming a chamber space, wherein a first opening is formed on the chamber wall. The osmosis membrane is disposed on the first opening to allow the lymph permeating into the chamber space of the chamber, wherein the osmosis membrane may be made of polymer molecules having biocompatibility. Herein, the osmosis membrane of the present invention may be designed in two ways, one is designed, in principle, with electrical properties being negative charged mainly, and another one is not specifically designed with electrical properties. Preferably, the osmosis membrane is made of polymer molecular materials having negative charges.

In addition, one end of the first catheter connects to the chamber space of the chamber, and the other end of the first catheter connects with a vein (about 2-5 mm in diameter) to allow the chamber space of the chamber connecting to the vein. The second sensing element receives a radio wave emitted by a first sensing element of a power supply device to emit an electrical signal. Furthermore, a pump is disposed in the chamber and electrically connects to the second sensing element, wherein the electrical signal emitted by the second sensing element drives the pump to generate a negative pressure in a range between 10 mmHg and 100 mmHg, preferably between 10 mmHg and 50 mmHg, more preferably between 10 mmHg and 30 mmHg, and most preferably between 10 mmHg and 15 mmHg, between 30 mmHg and 50 mmHg or between 50 mmHg and 80 mmHg in the chamber space to make the pressure inside of the chamber space lower than the pressure outside of the chamber space (i.e., tissue pressure), thereby guiding the lymph to permeate into the chamber space from the outside of the chamber space, and the lymph was drained to the vein via the first catheter to achieve the effect of remove the lymph between the tissues.

The aforementioned osmosis membrane may be made of polymer molecular materials having negative charges for the reason that fibroblasts would attach to the osmosis membrane and form a biomembrane when using an electrically neutral membrane, resulting in the clogging of the osmosis membrane. In respect of using an osmosis membrane having positive charges, bacteria may attach to the osmosis membrane in the case of infection, resulting in the clogging of the osmosis. Therefore, the osmosis membrane is most preferably made of polymer molecular materials having negative charges to prevent the osmosis membrane from clogging, which results in the requirement of surgery for replacing the osmosis membrane. If an osmosis membrane is not designed for the electric properties, there are several ways to solve such problems. For example, the minimal incision surgery may be performed at an appropriate location to replace the osmosis membrane during the outpatient surgery; or, we may use the silicone film on the surface to locally inject drugs that can prevent the osmosis membrane from the clogging, such as anticoagulants, fibroblast antibodies, and preventive or therapeutic antibiotics.

In the apparatus for draining lymph into veins of the present invention, it may further comprise a power supply device, wherein the power supply device may comprise the first sensing element, the first sensing element emits the radio wave, and the radio wave may be received by the second sensing element of the drainage device, thereby supplying the necessary power to the drainage device in a wireless manner. However, the present invention is not limited thereto.

In the apparatus for draining lymph into veins of the present invention, the power supply device may further comprise a power supply element electrically connected with the first sensing element for supplying power to the first sensing element. However, the present invention is not limited thereto. Herein, the power supply element may be a capacitor, a battery or any suitable energy storage device. The power supply is subject to no particular limitation, provided that it can supply electrical energy as required by the first sensing element. In addition, the power supply element is replaceable, but the present invention is not limited thereto. In the apparatus for draining lymph into veins of the present invention, the first sensing element and the second sensing element may be an induction coil respectively, but the present invention is not limited thereto. The first sensing element and the second sensing element may be any electromagnetic induction elements.

In the apparatus for draining lymph into veins of the present invention, the pump may comprise an inlet end and an outlet end, and the lymph permeating into the chamber space of the chamber is drained from the inlet end to the outlet end and drained to the vein via the first catheter. However, the present invention is not limited thereto.

In one aspect of the present invention, the pump may be disposed in the chamber space of the chamber, a second opening may be further formed on the chamber wall of the chamber, and the outlet end is connected with the second opening. Accordingly, the outlet end of the pump can directly guide the lymph in the chamber space of the chamber to the second opening connected to the outlet end, thereby achieving the purpose of draining the lymph from the outside of the apparatus for draining lymph into veins to the vein. However, the present invention is not limited thereto.

In another aspect of the present invention, the pump may be disposed outside of the chamber, a second opening may be further formed on the chamber wall of the chamber, the inlet end may be connected with the second opening, and the outlet end may be connected to the first catheter. The design of disposing the pump outside of the chamber allows the pump being detachable, and the pump therefore may be a detachable pump. If necessary, the pump may be changed to achieve the better effect of draining the lymph. However, the present invention is not limited thereto.

In a further aspect of the present invention, the pump may further comprise a second catheter, a second opening may be further formed on the chamber wall of the chamber, and the inlet end is connected with the second opening by the second catheter. Accordingly, one end of the second catheter would connect to the second opening and the other end would connect to the inlet end of the pump, so that the pump does not need to be disposed adjacent to the drainage device. The separation of the pump and chamber can reduce the volume of the drainage device, thereby reducing the discomfort of the patient at the implantation site. However, the present invention is not limited thereto. In addition, in order to respond to the lymphatic drainage conditions of different tissues, the pump of the present invention may be designed as a pressure-adjustable pump, a remote-controllable pump, or a wirelessly-controlled pump, such as a micropump, peristaltic micropump or manual micropump. However, the present invention is not limited thereto. By controlling the pump pressure and speed, the effect of controlling lymph drainage is achieved.

In the apparatus for draining lymph into veins of the present invention, the drainage device may further comprise an anti-reflux valve, and the anti-reflux valve may be, for example, but not limited to, a single plate type anti-reflux valve or a dual plate type anti-reflux valve. The anti-reflux valve may be disposed on at least one of the second opening, first catheter, and second catheter. For example, a dual plate type anti-flux forms two pieces of semi-curved elastic sheets on the inner wall of the catheter. When the lymph flows to the vein, it can pass through the anti-reflux valve 26, whereas the venous blood would be stopped by the anti-reflux valve 26 when the venous blood flows back from the vein. Thereby, it prevents the apparatus for draining lymph into veins of the present invention from the occurrence of the backflow of venous blood during the drainage process, so that it increases the lymph drainage effect of the apparatus for draining lymph into veins of the present invention.

The drainage device of the apparatus for draining lymph into veins of the present invention can be implanted into lymphedema tissue through surgical operations, minimally invasive surgery, and the like. In order to ensure that the drainage device of the present invention will not be rejected by the body, the periphery of the drainage device of the present invention may be covered with a biocompatible material, or made of a biocompatible material. However, the present invention is not limited thereto. Herein, all biocompatible materials approved by the FDA may be applied to the present invention, wherein the biocompatible material may be selected from the group consisting of silicone, silicone rubber, polyethylene, tetrafluoroethylene, poly-amino acid ester, polyurethane, polydimethylsiloxane, polylactic acid, polyglycolic acid, niobium-titanium-zirconium *β* alloy, titanium alloy metal, gold, silver, cobalt-chromium-molybdenum alloy, and poly (hydroxyethyl methacrylate). However, the present invention is not limited thereto. In addition, because the second sensing element of the drainage device can receive the radio wave emitted by the first sensing element of the power supply device and emit an electrical signal as the power source of the pump, the drainage device may be implanted into the subcutaneous tissue alone, and the power supply device may supply power through the skin. However, the present invention is not limited thereto.

In the apparatus for draining lymph into veins of the present invention, the lymph between tissues permeates into the chamber space of the chamber mainly by the negative pressure environment generated in the chamber space of the chamber, and the lymph in the chamber space is drained, by pump, into the vein connected to the first catheter, thereby achieving drainage. Since the first catheter is connected to the vein, its diameter should be smaller than that of the vein. Therefore, in the present invention, the diameter of the first catheter may ranges from 1 mm to 5 mm, preferably from 2 mm to 5 mm, and most preferably from 3 mm to 5 mm. However, the present invention is not limited thereto. Furthermore, the vein may be bundled and connected with the first catheter by a non-absorbable surgical thread.

Furthermore, the general size of protein is about 20 nm, the size of virus ranges from about 50 nm to hundreds of nanometers, and the size of bacteria is about thousands of nanometers. However, the size of albumin associated with lymphedema is about 3.8 nm. Therefore, in the apparatus for draining lymph into veins of the present invention, the pore size of the osmosis membrane used preferably allows the albumin which causes lymphedema passing through, whereas it prevents the smallest pathogens and viruses from passing. Therefore, the pore size of the osmosis membrane of the apparatus for draining lymph into veins of the present invention may be less than 50 nm, preferably between 10 nm and 50 nm, more preferably between 20 nm and 50 nm. However, the present invention is not limited thereto.

In addition, the apparatus for draining lymph into veins of the present invention may get clogged due to the mixing of protein tissues, blood cells, platelets or other substances. Therefore, the drainage device of the present invention may further comprise a drug injection silicone film disposed on the chamber to allow the drug injection silicone film injecting a drug into the chamber space of the chamber. However, the present invention is not limited thereto. Through this silicone film, skin-injected anticoagulant (such as heparin), related drugs (such as fibroblast antibody), or antibiotics are provided to reduce the blood clotting and clogging in the whole lymph drainage apparatus, or reduce the possibility of infection.

In addition, in order to facilitate the implantation of the lymph drainage apparatus of the present invention, the drainage device in the apparatus for draining lymph into veins of the present invention may further comprise a fixing ring disposed on an external wall of the chamber wall of the chamber, thereby fixing the drainage device on the body tissue using surgical thread. However, the present invention is not limited thereto. The number of the fixing ring may be 2-6, preferably 3-6, more preferably 4-6. The number of the fixing ring is subject to no particular limitation, provided that it can fix the drainage device in the body. The shape of the fixing ring is subject to no particular limitation, and it may be a hollow semicircular ring structure. In addition, the shape of the lymph drainage apparatus is subject to no particular limitation, and preferably is circular, oval, or the like. Secondly, the size of the apparatus of the present invention may be adjusted according to needs. Since it is better for patients who need lymphatic drainage to use apparatus having volume as little as possible, the preferable size of the drainage device is preferably less than 5 cm in diameter X 2 cm in height, more preferably between 4 cm X 1.5 cm, most preferably between 3 cm X 1 cm. However, the present invention is not limited thereto.

Other objects, advantages, and novel features of the invention will become more apparent from the following detailed description when taken in conjunction with the accompanying drawings.

### BRIEF DESCRIPTION OF THE DRAWINGS

FIG. 1 shows a partial schematic diagram of an apparatus for draining lymph into veins according to Embodiment 1 of the present invention.
FIG. 2 shows a schematic diagram of implantation of an apparatus for draining lymph into veins according to Embodiment 1 of the present invention.
FIG. 3 shows a schematic diagram of an apparatus for draining lymph into veins according to Embodiment 1 of the present invention.
FIG. 4 shows a schematic diagram of an apparatus for draining lymph into veins according to Embodiment 2 of the present invention.
FIG. 5 shows a schematic diagram of an apparatus for draining lymph into veins according to Embodiment 3 of the present invention.
FIG. 6 shows a schematic diagram of an apparatus for draining lymph into veins according to Embodiment 4 of the present invention.

### DETAILED DESCRIPTION OF THE PREFERRED EMBODIMENT

Different embodiments of the present invention are provided in the following description. These embodiments are meant to explain the technical content of the present invention, but not meant to limit the scope of the present invention. A feature described in an embodiment may be applied to other embodiments by suitable modification, substitution, combination, or separation.

It should be noted that, in the present specification, when a component is described to have an element, it means that the component may have one or more of the elements, and it does not mean that the component has only one of the element, except otherwise specified.

Moreover, in the present specification, the ordinal numbers, such as "first" or "second", are used to distinguish a plurality of elements having the same name, and it does not mean that there is essentially a level, a rank, an executing order, or an manufacturing order among the elements, except otherwise specified. A "first" element and a "second" element may exist together in the same component, or alternatively, they may exist in different components, respectively. The existence of an element described by a greater ordinal number does not essentially means the existent of another element described by a smaller ordinal number.

Herein, except otherwise specified, when it comes to feature A "or" or "and/or" feature B, it refers to the presence of A alone, B alone, or A and B exist at the same time. When it comes to feature A "and" feature B, it means that A and B exist at the same time. The term "comprise", "include", "have ", or "contain" means including but not limited thereto.

In addition, the term such "on", "under" or "between" used herein is only used to describe the relative position of multiple elements, and it can be extended to comprise translation, rotation or reflection during interpretation.

Moreover, in the present specification, when an element is described to be arranged "on" another element, it does not essentially means that the elements contact the other element, except otherwise specified. Such interpretation is applied to other cases similar to the case of "on".

Moreover, in the present specification, the terms, such as "preferably" or "advantageously", are used to describe an optional or additional element or feature, and in other words, the element or the feature is not an essential element, and may be ignored in some embodiments.

### Embodiment 1

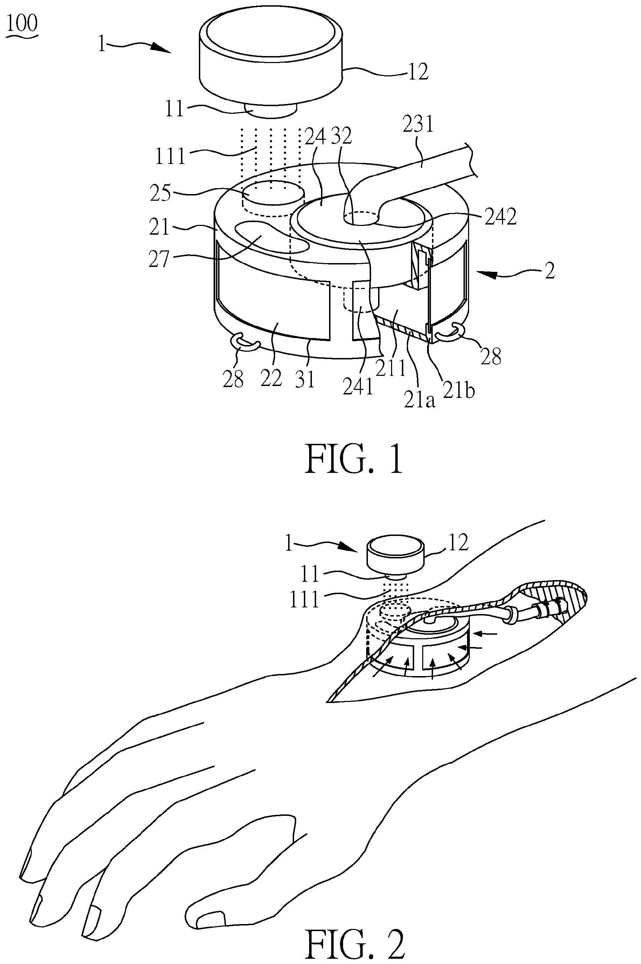

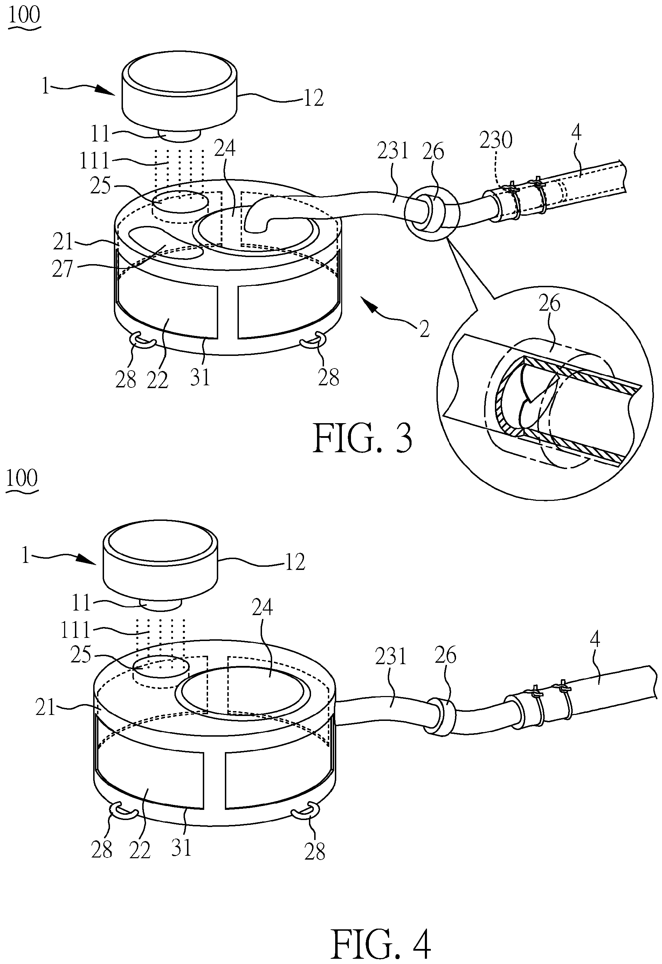

FIG. 1 shows a partial schematic diagram of an apparatus for draining lymph into veins 100 according to Embodiment 1 of the present invention. FIG. 2 shows a schematic diagram of implantation of an apparatus for draining lymph into veins 100 according to Embodiment 1 of the present invention. FIG. 3 shows a schematic diagram of an apparatus for draining lymph into veins 100 according to Embodiment 1 of the present invention.

As shown in FIG. 1 to FIG. 3, the apparatus for draining lymph into veins 100 of this embodiment comprises a power supply device 1 and a drainage device 2, wherein the power supply device 1 comprises a first sensing element 11 and a power supply element 12, the power supply element 12 is electrically connected to the first sensing element 11 to supply power to the first sensing element 11. The first sensing element 11 is used to emit a radio wave 111 and is an induction coil. However, the first sensing element 11 is subject to no particular limitation, provided that it can perform electromagnetic induction. In this embodiment, the power supply element 12 is a battery. However, the power supply element 12 is not limited thereto, and it can be a capacitor or any suitable energy storage device.

In addition, the drainage device 2 is implanted into the subcutaneous tissue and comprises a chamber 21, a osmosis membrane 22, a first catheter 231, a pump 24, a second sensing element 25, a drug injection silicone film 27, and a fixing ring 28, wherein the chamber 21 comprises a chamber wall 21a forming a chamber space 211, a first opening 31 and a second opening 32 are formed on the chamber wall 21a, the first opening 31 serves as an inlet for lymph, and the second opening 32 serves as an outlet for lymph. In this embodiment, the shape of the drainage device 2 is circular, but it is not limited thereto. The shape of the drainage device 2 can also be any suitable shape such as an ellipse. In one embodiment, the size (diameter) of the drainage device 2 is 3 cm, but it is not limited thereto. The size can be less than 5 cm, more preferably 1 to 5 cm, and most preferably 1 to 3 cm. In addition, the periphery of the drainage device 2 can be covered with a biocompatible material, or made of a biocompatible material, and the drainage device 2 is, for example, but not limited to, covered with polyurethane. Furthermore, the biocompatible material is selected from the group consisting of silicone, silicone rubber, polyethylene, tetrafluoroethylene, poly-amino acid ester, polyurethane, polydimethylsiloxane, polylactic acid, polyglycolic acid, niobium-titanium-zirconium *β* alloy, titanium alloy metal, gold, silver, cobalt-chromium-molybdenum alloy, and poly (hydroxyethyl methacrylate).

Secondly, the osmosis membrane 22 is disposed on the first opening 31 to allow the lymph permeating into the chamber space 211 of the chamber 21, wherein the osmosis membrane is made of polymer molecules having biocompatibility with a pore diameter between 20 nm and 50 nm in this embodiment. However, the osmosis membrane 22 is subject to no particular limitation, and it can be made of biocompatible polymer molecules, or biocompatible polymer molecules that are not specifically designed for electrical properties. Also, the pore size of the osmosis membrane 22 can be less than 50 nm, preferably between 10 nm and 50 nm, more preferably between 20 nm and 50 nm. One end of the first catheter 231 is connected to the chamber space 211 of the chamber 21, and the other end of the first catheter 231 is connected to the vein 4 (about 2-5 mm in diameter), so that the chamber space 211 of the chamber 21 is connected with the vein 4, wherein the diameter of the first catheter 231 should be smaller than that of the vein 4, and the diameter is 1.5 mm in one embodiment. However, it is not limited thereto. The diameter of the first catheter 231 can be in a range between 1 mm and 5 mm, preferably between 2 mm to 5mm, most preferably 3 mm to 5 mm, and the vein can be bundled and connected with the first catheter 231 by a non-absorbable surgical thread.

Furthermore, the second sensing element 25 is disposed on the chamber 21 of the drainage device 2 and is an induction coil (but not limited thereto) to receive the radio wave 111 emitted by the first sensing element 11 of the power supply device 1, thereby emitting an electrical signal to the pump 24. The pump 24 comprises an inlet end 241 and an outlet end 242, the pump 24 is disposed in the chamber space 211 of the chamber 21, and is connected to the second sensing element 25. The pump 24 is driven by the electrical signal emitted by the second sensing element 25 to generate a negative pressure in the chamber space 211, such that it makes the pressure inside of the chamber space 211 lower than the pressure outside of the chamber spacec211, thereby guiding the lymph to pass through the osmosis membrane 22 and permeate into the chamber space 211 from the outside of the chamber space 211. The lymph permeating to the chamber space 211 of the chamber 21 is drained from the inlet end 241 to outlet end 242 of the pump 24. Since the outlet end 242 of the pump 24 is connected with the second opening 32 formed on the chamber wall 21a, the lymph is drained to the vein 4 through the first catheter 231 to achieve the effect of lymphatic drainage. Herein, the negative pressure generated by the pump 24 in the chamber space 211 is 25 mmHg, but it is not limited thereto. The negative pressure can be in a range between 10 mmHg and 100 mmHg, preferably between 10 mmHg and 50 mmHg, more preferably between 10 mmHg and 30 mmHg, and most preferably between 10 mmHg and 15 mmHg, between 30 mmHg and 50 mmHg, or between 50 mmHg and 80 mmHg. In addition, the pump 24 is a micropump (but not limited thereto), and can also be a peristaltic micropump or a manual micropump.

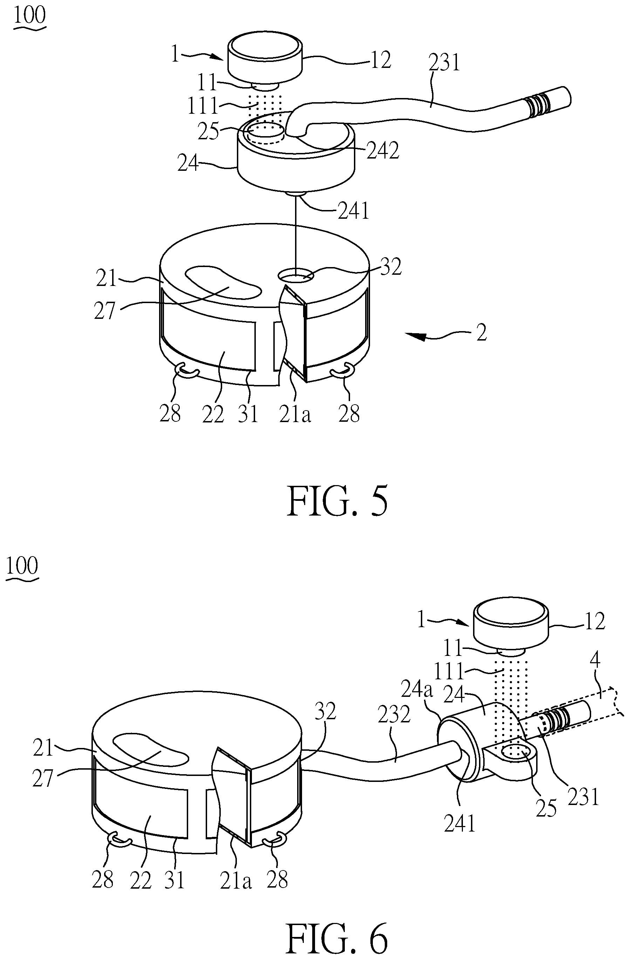

The drug injection silicone film 27 is disposed in the chamber 21 of the drainage device 2, so that the drug is injected from the drug injection silicone film 27 into the chamber space 211 of the chamber 21. However, the present invention is not limited thereto. The fixing ring 28 is disposed on an external wall 21b of the chamber wall 21a of the chamber 21 of the drainage device 2 to facilitate the fixing of the drainage device 2 with a surgical thread in the body. In this embodiment, the number of the fixing ring 28 is two and the fixing ring 28 has a hollow semicircular ring structure. However, the present invention is not limited thereto. The number of the fixing ring 28 can be 2-6, preferably 3-6, more preferably 4-6. The anti-reflux valve 26 is disposed on the first catheter 231 (but not limited thereto). In one embodiment, the anti-reflux valve 26 can also be disposed on the second opening 32 formed on the chamber wall 21a, or disposed on the second catheter 232 (as shown in FIG. 6). The function of the anti-reflux valve 26 is similar to the function of a heart valve. For example, the dual plate valve 26 forms two pieces of semi-curved elastic sheets on the inner wall of the catheter. When the lymph flows to the vein, it can pass through the anti-reflux valve 26, whereas the venous blood would be stopped by the anti-reflux valve 26 when the venous blood flows back from the vein. Thereby, it prevents the drained lymph from flowing back to the chamber 211, which affects the effect of lymph drainage. Herein, the anti-reflux valve 26 is a dual plate or three plate type anti-reflux valve, and the material thereof is an elastic material of silicone having biocompatibility. However, the present invention is not limited thereto.

In addition, the method for connecting the first catheter 231 and the vein 4 in this embodiment is that connect the first catheter 231 to the vein 4 through a groove 230 at the other end of the first catheter 231, and then use surgical threads to fix the first catheter 231 and the vein 4, allowing the permeated lymph in the apparatus for draining lymph into veins 100 of the present embodiment being guided to flow into the vein 4.

The operation of the apparatus for draining lymph into veins 100 of the present embodiment is described as follows. First, the power supply element 12 of the power supply device 1 supplies power to the first sensing element 11, so that the first sensing element 11 emits the radio wave 111. The emitted radio wave 111 can be received by the second sensing element 25 of the drainage device 2, and then an electrical signal is set out after conversion. Since the pump 24 is connected to the second sensing element 25, the converted electrical signal can drive the pump 24 of the drainage device 2. Therefore, the apparatus for draining lymph into veins 100 of the present embodiment can be driven in a wireless manner, without an additional power supply arranged in the drainage device 2. Then, the pump 24 generates a negative pressure in the chamber space 211 of the chamber 21. In other words, it makes the pressure inside of the chamber space 211 lower than the pressure outside of the chamber space 211, that is, the pressure in the chamber space 211 is lower than the pressure of tissue pressure outside, thereby guiding the lymph to permeate into the chamber space 211 of the chamber 21 from the outside. Further, the chamber space 211 of the chamber 21 is connected with the vein 4 through the first catheter 231 connecting the pump 24, thereby draining the lymph back to the vein 4 to ameliorate lymphedema and achieving the effect of removing the lymph between tissues. In addition, anticoagulant drugs, antibodies, or antibiotics can be injected into the chamber space 211 through the drug injection silicone film 27 according to actual needs.

### Embodiment 2

FIG. 4 shows a schematic diagram of an apparatus for draining lymph into veins according to this embodiment of the present invention.

As shown in FIG. 4, the configuration of this embodiment is similar to that of Embodiment 1, so that it has similar features and effects as Embodiment 1. Therefore, such descriptions are omitted. The difference from Embodiment 1 lies in the first catheter 231 being disposed on the lateral side of the chamber 21. In addition, the apparatus for draining lymph into veins 100 does not have a drug injection silicone film 27 (as shown in FIG. 3). In addition, as long as it is reasonable, the detailed features of the embodiments can also be combined arbitrarily.

### Embodiment 3

FIG. 5 shows a schematic diagram of an apparatus for draining lymph into veins 100 according to Embodiment 3 of the present invention.

As shown in FIG. 1 and FIG. 5, the configuration of this embodiment is similar to that of Embodiment 1, so that it has similar features and effects as Embodiment 1. Therefore, such descriptions are omitted. The differences from Embodiment 1 are the lack of anti-reflux valve 26 (as shown in FIG. 3) and the positions of the pump 24 and the second sensing element 25. In Embodiment 1, the pump 24 is disposed in the chamber 21 and the second sensing element 25 is also disposed in the chamber 21 directly. However, in the present embodiment, the pump 24 is disposed outside of the chamber 21 and adjacent to the chamber 21, in which the pump 24 is a micropump (but not limited thereto); the inlet end 241 of the pump 24 is connected with the second opening 32 formed on the chamber wall 21a; the outlet end 242 of the pump 24 is connected with the first catheter 231; and, the second sensing element 25 is directly disposed in the chamber 24 to save the connecting wire between the second sensing element 25 and the pump 24. As mentioned above, the design of disposing the pump 24 outside of the chamber 21 makes the pump 24 detachable, and the use of the pump 24 can be adjusted according to different needs. For example, the pump 24 is a detachable pump or designed for wireless control. The power and speed of the pump 24 can be adjusted to adjust the speed of the lymph drainage from the tissues to the vein 4 (as shown in FIG. 4). However, the present invention is not limited thereto. In addition, as long as it is reasonable, the detailed features of each embodiment can also be combined arbitrarily.

### Embodiment 4

FIG. 6 shows a schematic diagram of an apparatus for draining lymph into veins 100 according to Embodiment 4 of the present invention.

As shown in FIG. 1 and FIG. 6, the configuration of this embodiment is similar to that of Embodiment 3, so that it has similar features and effects as Embodiment 3. Therefore, such descriptions are omitted. The differences from Embodiment 3 are the lack of anti-reflux valve 26 (as shown in FIG. 3) and the arrangements of the pump 24, first catheter 231, second catheter 232 and second sensing element 25. The pump 24 of Embodiment 3 is disposed outside and adjacent to the chamber 21. However, the pump 24 of the present embodiment has the second catheter 232, in which one end of the second catheter 232 is connected with the second opening 32 formed on the chamber wall 21a and the other end is connected with the inlet end 241 of the pump 24. In other words, the inlet end 241 of the pump 24 is connected to the second opening 32 on the chamber wall 21a through the second catheter 232, the first catheter 231 connects to the outlet end 242 of the pump 24 (as shown in FIG.5), and the first catheter 231 is connected with the vein 4. The separation design of the pump 24 and chamber 21 makes the pumps 24 become detachable.

Since the pump 24 of this embodiment is designed in a separating manner, it can be connected in series according to different needs, thereby achieving the negative pressure required by the chamber space 211 of the chamber 21 to facilitate lymph drainage. Furthermore, this separating pump can be replaced with a manual micropump, the pump 24 can be supplied with power manually in addition to electrically. Secondly, the second sensing element 25 is disposed on the external wall 24a of the pump 24 so as to receive the radio wave 111 from the first sensing element 11 of the power supply element 1. Furthermore, the second catheter 232 of the present embodiment is disposed on the lateral side of the chamber 21, thereby reducing the discomfort of the lymphedema affected part of the patient when the apparatus for draining lymph into veins 100 of the present invention is implanted. In addition, as long as it is reasonable, the detailed features of the embodiments can also be combined arbitrarily.

Although the present invention has been explained in relation to its preferred embodiment, it is to be understood that many other possible modifications and variations can be made without departing from the spirit and scope of the invention as hereinafter claimed.

## Claims

1. An apparatus for draining lymph into veins (100), comprising:
a drainage device (2), comprising:
a chamber (21), comprising a chamber wall (21a) forming a chamber space (211), wherein a first opening (31) is formed on the chamber wall (21a);
an osmosis membrane (22), disposed on the first opening (31) to allow the lymph permeating into the chamber space of the chamber (211), the pore size of said osmosis membrane (22) being preferably in a range between 20 nm and 50 nm;
a first catheter (231), wherein one end of the first catheter (231) connects to the chamber space (211) of the chamber (21), and the other end of the first catheter (231) connects with a vein (4) to allow the chamber space (211) of the chamber (21) connecting to the vein (4), the diameter of said first catheter (231) being preferably in a range between 1 mm and 2 mm;
a second sensing element (25), receiving a radio wave (111) emitted by a first sensing element (11) of a power supply device (1) to emit an electrical signal; and
a pump (24), disposed in the chamber (21) and connecting to the second sensing element (25), wherein the electrical signal emitted by the second sensing element (25) drives the pump (24) to generate a negative pressure in the chamber space (211) to make the pressure inside the chamber space (211) lower than the pressure outside the chamber space (211), thereby guiding the lymph to permeate into the chamber space (211) from the outside of the chamber space (211), and the lymph was drained to the vein (4) via the first catheter (231).

2. The apparatus of claim 1, further comprising the power supply device (1), wherein the power supply device (1) comprises the first sensing element (11) and the first sensing element (11) emits the radio wave (111).

3. The apparatus of claim 2, wherein the power supply device (1) further comprises a power supply element (12) electrically connected with the first sensing element (11) for supplying power to the first sensing element (11).

4. The apparatus of claim 2, wherein the first sensing element (11) and the second sensing element (25) are respectively an induction coil.

5. The apparatus of claim 1, wherein the pump (24) comprises an inlet end (241) and an outlet end (242), and the lymph permeating into the chamber space (211) of the chamber (21) is drained from the inlet end (241) to the outlet end (242) and drained to the vein (4) via the first catheter (231).

6. The apparatus of claim 5, wherein the pump (24) is disposed in the chamber space (211) of the chamber (21), a second opening (32) is further formed on the chamber wall (21a) of the chamber (21), and the outlet end (242) is connected with the second opening (32).

7. The apparatus of claim 5, wherein the pump (24) is disposed outside of the chamber (21), a second opening (32) is further formed on the chamber wall (21a) of the chamber (21), the inlet end (241) is connected with the second opening (32), and the outlet end (242) is connected to the first catheter (231).

8. The apparatus of claim 7, wherein the pump (24) further comprises a second catheter (232), and the inlet end (241) is connected with the second opening (32) by the second catheter (232), the pump (24) being preferably a detachable pump.

9. The apparatus of claim 1, wherein the pump (24) is a micropump, a peristaltic micropump, or a manual micropump.

10. The apparatus of claim 1, wherein the drainage device (2) further comprises an anti-reflux valve (26) disposed in the first catheter (231).

11. The apparatus of claim 1, wherein the drainage device (2) is implanted in subcutaneous tissue.

12. The apparatus of claim 1, wherein the periphery of the drainage device (2) is covered with a biocompatible material preferably selected from the group consisting of silicone, silicone rubber, polyethylene, tetrafluoroethylene, poly-amino acid ester, polyurethane, polydimethylsiloxane, polylactic acid, polyglycolic acid, niobium-titanium-zirconium *β* alloy, titanium alloy metal, gold, silver, cobalt-chromium-molybdenum alloy, and poly (hydroxyethyl methacrylate).

13. The apparatus of claim 1, wherein the osmosis membrane (22) is made of polymer molecular materials having negative charges.

14. The apparatus of claim 1, wherein the negative pressure generated by the pump (24) in the chamber space (211) is in a range between 10 mmHg and 30 mmHg.

15. The apparatus of claim 1, wherein the drainage device (2) further comprises a drug injection silicone film (27) disposed on the chamber (21) to allow the drug injection silicone film (27) injecting a drug into the chamber space (211) of the chamber (21), said drainage device (2) optionally further comprising a fixing ring (28) disposed on an external wall (21b) of the chamber wall (21a) of the chamber (21).
